(19) Europäisches Patentamt
European Patent Office
Office européen des brevets

(11) Veröffentlichungsnummer: **0 398 018 B1**

(12) # EUROPÄISCHE PATENTSCHRIFT

(45) Veröffentlichungstag der Patentschrift: **01.02.95**

(21) Anmeldenummer: **90106741.3**

(22) Anmeldetag: **09.04.90**

(51) Int. Cl.6: **C07D 417/06**, C07D 417/12, C07D 401/06, C07D 421/06, G03F 7/029, //(C07D417/06, 277:00,251:00),(C07D417/12, 277:00,251:00),(C07D401/06, 251:00,215:00),(C07D421/06, 263:00,251:00)

(54) **Verfahren zur Herstellung von lichtempfindlichen Bis-trichlor-methyl-s-triazinen.**

(30) Priorität: **18.04.89 DE 3912652**

(43) Veröffentlichungstag der Anmeldung: **22.11.90 Patentblatt 90/47**

(45) Bekanntmachung des Hinweises auf die Patenterteilung: **01.02.95 Patentblatt 95/05**

(84) Benannte Vertragsstaaten: **CH DE FR GB LI**

(56) Entgegenhaltungen:
**EP-A- 0 135 348**
**EP-A- 0 135 863**
**DE-A- 2 243 621**
**DE-A- 2 717 778**
**DE-A- 2 718 259**
**DE-A- 3 726 001**
**US-A- 2 475 949**

**ORGANIC SYNTHESES, COLL. Band III, 1955, Seiten 595, 596; H.W.J. CRESSMAN: "1-Methyl-2-imino-beta-naphthothiazoline"**

(73) Patentinhaber: **HOECHST AKTIENGESELLSCHAFT**
**D-65926 Frankfurt (DE)**

(72) Erfinder: **Wilczak, Wojciech, Dr.**
**205 Ten Street,**
**Apartment 4L**
**Jersey City,**
**New Jersey 07302 (US)**

**EP 0 398 018 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 4,6-Bis-trichlormethyl-s-triazinen, die in 2-Stellung substituiert sind.

Verbindungen der genannten Gattung sind als Initiatoren für verschiedene photochemische Reaktionen bekannt. Sie werden einerseits eingesetzt, um die bei Einwirkung von aktinischer Strahlung entstehenden Radikale zur Auslösung von Polymerisationsreaktionen oder Farbveränderungen auszunutzen, oder andererseits, um durch die freigesetzte Säure Folgereaktionen zu bewirken.

In der DE-C 22 43 621 (= US-A 3 954 475) werden styryl-substituierte Trichlormethyl-s-triazine beschrieben, die eine Reihe von vorteilhaften Eigenschaften haben. Nachteilig ist ihre relativ komplizierte Herstellung.

In der DE-C 27 18 259 (= US-A 4 189 323) werden 2-Aryl-4,6-bis-trichlormethyl-s-triazine mit mehrkernigen Arylgruppen beschrieben, die ähnlich vorteilhafte Eigenschaften, insbesondere eine hohe Lichtempfindlichkeit, aufweisen und die sich auf einfachere Weise herstellen lassen. Sie werden durch Cotrimerisierung von Trichloracetonitril mit aromatisch substituierten Acetonitrilen in für diese Art Reaktion recht hoher Ausbeute erhalten. Es entstehen jedoch stets auch in gewisser Menge Nebenprodukte, die von der gewünschten Verbindung abgetrennt werden müssen.

Aus der DE-B 27 17 778 sind ferner lichtempfindliche Gemische auf Basis ungesättigter Verbindungen oder polymerer Azide bekannt, die als Sensibilisatoren 2-Heteroylcarbonylmethylen-benzthiazole oder -benzselenazole enthalten.

In der DE-A 25 51 641 sind photopolymerisierbare Gemische beschrieben, die als Photoinitiatoren eine Kombination von bestimmten Trihalogenmethyl-s-triazinen mit Acylmethylenheterocyclen, z. B. Benzoylmethylen-benzthiazolinen enthalten.

Aus den EP-A 135 348 und 135 863 sind 1-Alkyl-2-carbonylmethylen-benzthiazole und ähnliche Heterocyclen als Photoinitiatoren bekannt, die an der Carbonylgruppe eine Trichlormethylphenylgruppe tragen.

Aus der DE-A 37 26 001 sind photopolymerisierbare Gemische bekannt, die als Photoinitiatoren Bis-trihalogenmethyl-s-triazinylbenzole enthalten, die weiter durch eine Aminogruppe substituiert sind. Die Empfindlichkeit der Gemische kann durch Zusatz von Sensibilisatoren, z. B. Michlers Keton oder Benzoylenethylenbenzthiazolen, gesteigert werden.

In der EP-A 137 452 werden 2-(Styrylphenyl)-4,6-bis-trichlormethyl-s-triazine als Photoinitiatoren und photolytisch anregbare Säurespender beschrieben.

In der älteren deutschen Patentanmeldung P 38 07 381.1 werden ähnliche Verbindungen mit Bis-trichlormethyl-s-triazingruppen vorgeschlagen, die als externe Substituenten an einem Aroylmethylenheterocyclus sitzen.

Die meisten dieser als Photoinitiatoren wirksamen Verbindungen werden auf verhältnismäßig komplizierten Synthesewegen hergestellt. Die wegen ihrer hohen Wirksamkeit häufig bevorzugten Trichlormethyl-s-triazinderivate werden praktisch alle auf dem oben erwähnten Weg der Cotrimerisierung von Trichloracetonitril mit anderen Nitrilen erhalten. Die neuere Entwicklung der Technik ist vor allem darauf gerichtet, bekannte gut wirksame oder ähnliche Verbindungen auf Synthesewegen zugänglich zu machen, bei denen weniger Nebenprodukte entstehen, deren umweltgerechte Entsorgung bekanntlich immer größere Probleme bereitet.

Aufgabe der Erfindung war es daher, lichtempfindliche Verbindungen vom Typ der substituierten Bis-trichlormethyl-s-triazine bereitzustellen, die eine ähnlich gute Wirksamkeit als Photoinitiatoren und Säurespender wie die bekannten Verbindungen gleichen Typs aufweisen, aber in einfacherer Weise und mit höheren Ausbeuten zugänglich sind.

Erfindungsgemäß werden lichtempfindliche Bis-trichlormethyl-s-triazine der allgemeinen Formel I

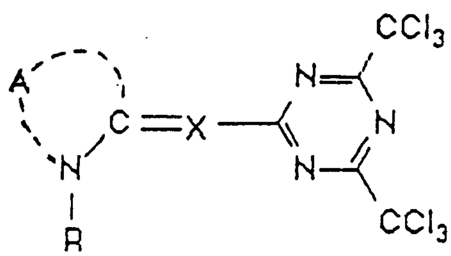

(I)

vorgeschlagen, worin

A die zur Vervollständigung eines 5- oder 6-gliedrigen heterocyclischen Rings erforderlichen Ringglieder, der substituiert sein oder einen ggf. substituierten ankondensierten Benzolring tragen kann,

R eine ggf. substituierte Alkylgruppe und

X CH oder N

bedeutet.

Erfindungsgemäß wird ferner ein lichtempfindliches Gemisch vorgeschlagen, das ein Bis-trichlormethyl-s-triazin (a) und eine Verbindung (b) enthält, die mit dem Lichtreaktionsprodukt des Triazins (a) unter Ausbildung eines Produktes zu reagieren vermag, das eine von (b) unterschiedliche Lichtabsorption oder Löslichkeit in einem Entwickler aufweist.

Das erfindungsgemäße Gemisch ist dadurch gekennzeichnet, daß das Triazin (a) eine Verbindung der Formel I gemäß vorstehender Definition ist.

Erfindungsgemäß wird schließlich auch ein Verfahren zur Herstellung einer Verbindung der Formel I vorgeschlagen, das darin besteht, daß man 2,4,6-Tris-trichlormethyl-s-triazin mit dem Salz einer quaternären N-heterocyclischen Base der Formel III oder einem heterocyclischen Imin der Formel IV

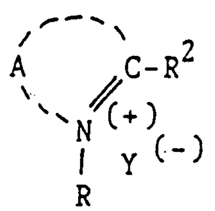 (III) 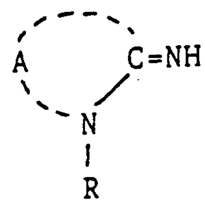 (IV)

umsetzt, wobei

$R^2$ eine Methyl- oder Aminogruppe ist,

$Y^{(-)}$ ein Anion ist und

A und R die oben angegebene Bedeutung haben.

In der allgemeinen Formel I bildet A 3 oder 4, vorzugsweise 3 Ringglieder. Von den dabei gebildeten Fünfringheterocyclen wird das Thiazolin bzw. Thiazolidin besonders bevorzugt. Der Ring kann, z. B. durch Alkyl-, Aryl-, Alkoxy-, Aryloxygruppen oder Halogenatome, substituiert sein. Er kann bevorzugt einen ankondensierten Benzol- oder Naphthalinkern, insbesondere einen Benzolkern, tragen, der selbst wiederum Substituenten gleicher Art wie der heterocyclische Ring tragen kann. Von den genannten Substituenten haben Alkyl- und Alkoxygruppen 1 bis 6 Kohlenstoffatome; ihre Ketten können durch Ethersauerstoffatome unterbrochen sein. Die aromatischen Substituenten haben 6 bis 10 Kohlenstoffatome. Als weitere Heteroatome in den Fünf- oder Sechsringheterocyclen kommen außer S noch Se, O oder N in Betracht.

R ist eine unsubstituierte oder substituierte Alkyl-gruppe. Als Substituenten kommen insbesondere Alkoxy-, Aryl-, Aryloxygruppen oder Halogenatome in Betracht, wobei Alkoxy- und Arylgruppen bevorzugt werden. Rein aliphatische Substituenten R können normalerweise 1 bis 6, solche mit aromatischen Anteilen 7 bis 10 Kohlenstoffatome enthalten. Beispiele für heterocyclische Gruppen der hier geeigneten Art sind z. B. in der EP-A 135 863 beschrieben.

X ist vorzugsweise eine CH-Gruppe.

Als Ausgangsstoff zur Herstellung der erfindungsgemäßen Verbindungen wird das Tris-trichlormethyl-s-triazin eingesetzt. Die Verbindung ist in bekannter Weise durch Trimerisieren von Trichloracetonitril in hohen, nahezu quantitativen Ausbeuten zugänglich (Bull. Chem. Soc. Jap. 42, 2924 (1969). Sie wird mit einer Verbindung einer der Formeln III oder IV umgesetzt, wobei unter Austritt von HCl und ggf. HY die Verbindung der Formel I gebildet wird. Die Umsetzung wird vorteilhaft in einem Lösemittel und in Gegenwart einer Base durchgeführt. Als Anionen Y der quaternären Base werden zweckmäßig solche von starken anorganischen oder organischen Säuren, z. B. von Halogenwasserstoffsäuren wie HCl, Schwefelsäure oder Sulfonsäuren, z. B. p-Toluolsulfonsäure, eingesetzt.

Als Lösemittel sind z. B. Toluol, Xylol, Benzol, Dimethylformamid, Diethylether, Diisopropylether, Methylenchlorid, Chloroform, Pyridin, Ethylacetat, Methanol, Ethanol, tert.-Butanol und Gemische derartiger Substanzen geeignet.

Beispiele für geeignete Basen sind tertiäre Amine wie Triethylamin, Dimethylbenzylamin, Diethylbenzylamin, N-Ethyldicyclohexylamin, N-Ethylpiperidin, N-Methylmorpholin, N-Ethylpyrrolidon, 1,8-Diazabicyclo-[5.4.0]undec-7-en, 1,4-Diazabicyclo[2.2.2]octan oder Pyridin.

Die Umsetzung erfolgt vorzugsweise bei mäßig erhöhter Temperatur, z. B. im Bereich von 20 bis 60 °C. In der Regel ist sie nach etwa 30 Minuten beendet. Auch die Ausbeute bei dieser Reaktionsstufe ist sehr hoch und meist nahezu quantitativ.

Geeignete quaternäre Salze der Formel III sind z. B. in J.Pharm.Soc.Jap. 1951, 71 und in Hamer, "Cyanine Dyes and Related Compounds", Interscience Publishers, 1964, S. 116-147 beschrieben. Geeignete heterocyclische Imine der Formel IV sind beschrieben in Cressman, Org. Synth. Coll. Vol. III, S. 595 (1955) und der US-A 2 475 949.

Die erfindungsgemäßen Verbindungen bilden unter Einwirkung von aktinischer Strahlung, besonders sichtbarem und UV-Licht, freie Radikale, die zur Einleitung chemischer Reaktionen, insbesondere von radikalisch initiierten Polymerisationen, befähigt sind. Sie spalten ferner bei Bestrahlung Halogenwasserstoff ab, durch den säurekatalysierte Reaktionen, z. B. die Spaltung von Acetalbindungen, oder Salzbildungen, z. B. Farbumschläge von Indikatorfarbstoffen, in Gang gesetzt werden können.

Die erfindungsgemäßen Verbindungen sind als Photoinitiatoren für photopolymerisierbare Schichten geeignet, die als wesentliche Bestandteile Monomere, Bindemittel und Initiatoren enthalten.

Für diese Anwendung brauchbare photopolymerisierbare Monomere sind bekannt und z. B. in den US-A 2 760 863 und 3 030 023 beschrieben.

Bevorzugte Beispiele sind Acryl- und Methacrylsäureester mehrwertiger Alkohole, wie Diglycerindiacrylat, Polyethylenglykoldimethacrylat, Acrylate und Methacrylate von Trimethylolethan, Trimethylolpropan und Pentaerythrit und von mehrwertigen alicyclischen Alkoholen. Mit Vorteil werden auch Umsetzungsprodukte von Diisocyanaten mit Partialestern mehrwertiger Alkohole eingesetzt. Derartige Monomere sind in den DE-A 20 64 079, 23 61 041 und 28 22 190 beschrieben.

Der Mengenanteil der Schicht an Monomeren beträgt im allgemeinen etwa 10 bis 80, vorzugsweise 20 bis 60 Gew.-%.

Als Bindemittel können eine Vielzahl löslicher organischer Polymerisate Einsatz finden. Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrig-alkalischen Entwicklern entwickeln lassen. Derartige Bindemittel können z. B. die folgenden Gruppen enthalten: -COOH, $-PO_3H_2$, $-SO_3H$; $-SO_2NH-$, $-SO_2NHSO_2-$ und $-SO_2-NH-CO-$.

Als Beispiele hierfür seien genannt: Maleinatharze, Polymerisate aus N-(p-Tolyl-sulfonyl)-carbaminsäure-($\beta$-methacryloyloxy-ethyl)ester und Mischpolymerisate dieser und ähnlicher Monomerer mit anderen Monomeren sowie Styrol-Maleinsäureanhydrid-Mischpolymerisate. Alkylmethacrylat-Methacrylsäure-Mischpolymerisate und Mischpolymerisate aus Methacrylsäure, Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:
Inhibitoren zur Verhinderung der thermischen Polymerisation der Monomeren,
Wasserstoffdonatoren,
die spektrale Empfindlichkeit derartiger Schichten modifizierende Stoffe,
Farbstoffe,
gefärbte und ungefärbte Pigmente,
Farbbildner,
Indikatoren,
Weichmacher usw.

Die Lichtempfindlichkeit der erfindungsgemäßen photopolymerisierbaren Gemische läßt sich durch Zusatz von Sensibilisatoren weiter steigern. Geeignete Sensibilisatoren sind z. B. Benzoin, Benzoinderivate, 9-Fluorenon, 9-Anthron, 9,10-Anthrachinon, Xanthon und deren Substitutionsprodukte, Thioxanthon, Benzil, Dibenzalaceton, p-Dimethylamino-phenylstyrylketon, Benzophenon, substituierte Benzophenone und insbesondere Michlers Keton. Weitere bevorzugte Sensibilisatoren sind Verbindungen der Formel II

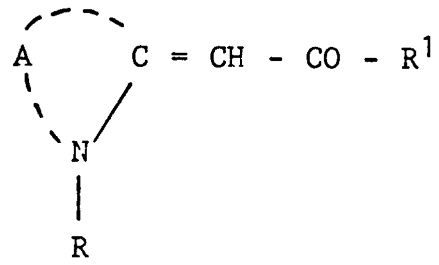

, (II)

die z. B. in der US-A 3 870 524 beschrieben sind. Darin haben A und R die oben im Zusammenhang mit Formel I angegebene Bedeutung. $R^1$ ist eine Alkyl-oder Arylgruppe. Ein bevorzugter Vertreter dieser Verbindungsklasse ist 2-Benzoylmethylen-3-methylnaphtho[1,2-d]thiazolin.

Die Sensibilisierung ist besonders wirksam bei Verbindungen der Formel I mit X = N.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Aufzeichnungsmaterial auf dem Reproduktionsgebiet. Als Anwendungsmöglichkeiten auf diesem Gebiet seien genannt: Kopierschichten für die photomechanische Herstellung von Druckformen für den Hochdruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von kopierten Schaltungen und für das Formteilätzen, anwendbar.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung gedruckter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen. Ebenso ist es für die Herstellung von Trockenresists geeignet.

Es ist im allgemeinen günstig, die Gemische während der Lichtpolymerisation dem Einfluß des Luftsauerstoffs weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Celluloseacetat, sowie Siebdruckträger, wie Perlongaze, geeignet.

Die lichtempfindlichen Verbindungen sind als Photoinitiatoren bereits in Konzentrationen von etwa 0,05 % des Gesamtfeststoffs der Masse wirksam, eine Erhöhung über 12 % ist im allgemeinen unzweckmäßig. Vorzugsweise werden Konzentrationen von 0,1 bis 8 % verwendet.

Weiterhin können die erfindungsgemäßen Verbindungen auch in solchen lichtempfindlichen Gemischen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Reaktionen auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säuren erfolgt, deren Anionen eine geringere Nukleophilie als Chlorid und Bromid besitzen, also als die Anionen der bei der Photolyse der neuen Verbindungen entstehenden Halogenwasserstoffsäuren, so härten doch Schichten, die aus Epoxyharzen und Novolaken bestehen, bei Belichtung in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farb-vorläufern, z. B. Leukoverbindungen, Farbbildung zu induzieren oder bathochrome Farbverschiebungen und -vertiefungen in Gemischen zu

bewirken, die Cyanin-, Merocyanin-oder Styrylfarbbasen enthalten. Auch kann z. B. in den in der DE-A 15 72 080 beschriebenen Gemischen, die Farbbase, N-Vinylcarbazol und einen Halogenkohlenwasserstoff enthalten, die Halogenverbindung Tetrabrommethan durch einen Bruchteil ihrer Menge an erfindungsgemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können.

Ein besonders bevorzugtes Anwendungsgebiet für die erfindungsgemäßen Verbindungen sind Gemische, die neben ihnen als wesentliche Komponente eine Verbindung mit mindestens einer durch Säure spaltbaren C-O-C-Gruppierung enthalten. Als durch Säure spaltbare Verbindungen sind in erster Linie zu nennen:

A) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können und

B) Polymerverbindungen mit wiederkehrenden Acetal- und/oder Ketalgruppierungen.

Durch Säure spaltbare Verbindungen des Typs A als Komponenten strahlungsempfindlicher Gemische sind in den DE-A 26 10 842 oder 29 28 636 ausführlich beschrieben; Gemische, die Verbindungen des Typs B enthalten, sind Gegenstand der DE-C 27 18 254.

Als durch Säure spaltbare Verbindungen sind z. B. auch die speziellen Aryl-alkyl-acetale und -aminale der DE-C 23 06 248 zu nennen, die durch die Photolyseprodukte der erfindungsgemäßen Verbindungen ebenfalls abgebaut werden.

Solche Gemische, in denen durch Einwirkung von aktinischer Strahlung mittelbar oder unmittelbar Moleküle in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden.

Die bei vielen Positiv-Aufzeichnungsmaterialien bewährten Novolak-Kondensationsharze haben sich als Zusatz auch bei der Anwendung der erfindungsgemäßen Verbindungen in Gemischen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55 - 85 Gew.-%.

Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann das lichtempfindliche Gemisch für spezielle Erfordernisse wie Flexibilität, Haftung und Glanz etc. außerdem noch Substanzen wie Polyglykole, Cellulose-Derivate wie Ethylcellulose, Netzmittel, Farbstoffe und feinteilige Pigmente sowie bei Bedarf UV-Absorber enthalten. Entwickelt wird vorzugsweise mit in der Technik üblichen wäßrig-alkalischen Entwicklern, die auch kleine Anteile organischer Lösemittel enthalten können, oder aber mit organischen Lösemitteln.

Die bereits im Zusammenhang mit den photopolymerisierbaren Gemischen aufgeführten Träger kommen ebenfalls für positiv arbeitende Aufzeichnungsmaterialien in Frage, zusätzlich die in der Mikroelektronik üblichen Silizium- und Siliziumdioxidoberflächen.

Die Menge der als Säurespender eingesetzten erfindungsgemäßen Verbindungen kann zwischen etwa 0,1 und 10 %, bezogen auf den Gesamtfeststoff, bevorzugt zwischen etwa 0,2 und 5 % liegen.

Bevorzugte Anwendung finden die eine der erfindungsgemäßen Verbindungen enthaltenden lichtempfindlichen Gemische bei der Herstellung von Druckformen, d. h. insbesondere Offset-, autotypischen Tiefdruck- und Siebdruckformen, in Photoresistlösungen und in sogenannten Trockenresists.

Die folgenden Beispiele dienen zur näheren Erläuterung der Erfindung; es folgt zunächst eine tabellarische Zusammenstellung von erfindungsgemäßen Verbindungen, die hergestellt und in lichtempfindlichen Gemischen eingesetzt wurden. In den Herstellungsbeispielen 1 und 2 wird anhand der Verbindungen 2 und 11 die Herstellungsweise erläutert. Daran schließen sich Beispiele für die Anwendung von einigen Verbindungen in lichtempfindlichen Gemischen an.

In den Beispielen sind die Mengen in Gew.-Teilen (Gt) angegeben. Prozent- und Mengenangaben sind, wenn nichts anderes angegeben ist, in Gewichtseinheiten zu verstehen.

## Tabelle

Verbindungen der allgemeinen Formel I

| 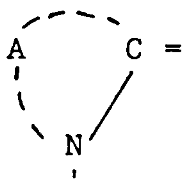 | R | X |
|---|---|---|
| 1.) Thiazolidin | $C_2H_5$ | CH |
| 2.) Benzthiazolin | " | " |
| 3.) Naphtho[1,2-d]thiazolin | $CH_3$ | " |
| 4.) Benzthiazolin | " | " |
| 5.) Benzthiazolin | Benzyl | " |
| 6.) Benzselenazolin | $CH_3$ | " |
| 7.) Chinolin | $C_2H_5$ | " |
| 8.) Thiazolidin | Benzyl | " |
| 9.) Benzthiazolin | Methoxyethyl | " |
| 10.) 6-Methyl-Benzselenazolin | $CH_3$ | " |
| 11.) Benzthiazolin | $CH_3$ | N |

Herstellungsbeispiel 1

3-Ethyl-2-(4,6-bis-trichlormethyl-s-triazin-2-yl-methylen)benzthiazolin (Verbindung 2)

Zu einer Suspension von 35 g (0,1 mol) 3-Ethyl-2-methyl-benzthiazolium-p-toluolsulfonat und 43 g (0,1 mol) Tris-trichlormethyl-s-triazin in 300 ml Toluol wurden tropfenweise 10 g (0,1 mol) Triethylamin innerhalb einer halben Stunde zugesetzt. Nach beendeter Zugabe wurde bis zum Abklingen der exothermen Reaktion noch eine halbe Stunde gerührt, das ausgefallene Produkt abfiltriert und getrocknet. Es wurden 40 g der Verbindung 2 in nahezu reiner Form erhalten. Durch Verdampfen des Toluols im Vakuum und Behandeln des festen Rückstands mit Ethanol wurden weitere 9 g erhalten. Nach Umkristallisieren aus 2-Methoxyethanol wurden 40 g (82 %) gelber Nadeln vom Schmelzpunkt 238 bis 241 °C erhalten.
$\lambda_{max}$ ($CHCl_3$) = 412 nm;
$^1H$ NMR ($CDCl_3$): 1,47 (t, 3H); 4,25 (q, 2H); 6,21 (s, 1H); 7,16 (m, 4H) ppm

| Analyse ($C_{15}H_{10}N_4SCl_6$) | | | | |
|---|---|---|---|---|
| | C | H | N | Cl |
| Ber.: | 36,68 | 2,05 | 11,41 | 43,30 |
| Gef.: | 36,4 | 1,9 | 11,3 | 43,1 |

Herstellungsbeispiel 2

3-Methyl-2-(4,6-bis-trichlormethyl-s-triazin-2-yl-imino)benzthiazolin (Verbindung 11)

Zu einer etwa 50 °C warmen Lösung von 4,3 g (0,01 mol) Tris-trichlormethyl-s-triazin und 1,7 g (0,01 mol) 3-methyl-2-imino-benzthiazolin in 50 ml Toluol wurden tropfenweise 1,1 g (0,01 mol) Triethylamin gegeben. Das Gemisch wurde eine halbe Stunde gerührt, das ausgefallene Produkt abfiltriert und getrocknet. Es wurden 3 g Rohprodukt erhalten; weitere 0,8 g wurden aus der Mutterlauge gewonnen. Die Rohausbeute an Verbindung 11 (3,8 g) betrug 79 %. Nach Umkristallisieren aus Ethanol wurden cremefarbige Kristalle vom Schmelzpunkt 251-253 °C erhalten.
$\lambda_{max}$ ($CHCl_3$) = 352 nm
$^1H$ NMR ($CDCl_3$): 4,07 (s, 3H); 7,47 (m, 4H)

| Analyse ($C_{13}H_7N_5SCl_6$): | | | | |
|---|---|---|---|---|
| | C | H | N | Cl |
| Ber.: | 32,66 | 1,48 | 14,65 | 44,5 |
| Gef.: | 32,5 | 1,3 | 14,5 | 44,4 |

Anwendungsbeispiel 1

Eine Lösung von
1,40 Gt des Polyacetals aus 2-Ethyl-butyraldehyd und Triethylenglykol,
4,70 Gt Kresol-Formaldehyd-Novolak, Schmelzbereich 105-120 °C,
0,23 Gt Verbindung 5 und
0,02 Gt Kristallviolettbase in
45 Gt Propylenglykolmonomethylether,
28 Gt Tetrahydrofuran und
20 Gt Dimethylformamid
wurde auf eine Platte aus elektrolytisch aufgerauhtem und anodisch oxydiertem Aluminium aufgeschleudert. Nach dem Trocknen hatte die lichtempfindliche Schicht ein Gewicht von 2,0 g/m$^2$. Die Platte wurde mit einer 5000-W-Metallhalogenidlampe 30 Sekunden im Kontakt unter einer Vorlage belichtet, die einen Stufenkeil mit Dichteinkrementen von 0,15 enthielt. Nach 10 Minuten Wartezeit wurde die Platte 1 Minute mit der folgenden Lösung entwickelt:
5,5 g Natriummetasilikat x 9 $H_2O$,
3,4 g Trinatriumphosphat x 12 $H_2O$,
0,4 g Natriumdihydrogenphosphat (wasserfrei),
90,7 g Wasser.
Es wurde ein positives Bild erhalten, das bis zur Stufe 4 frei entwickelt war.

Anwendungsbeispiel 2

Eine Lösung von
4,30 Gt Phenol-Formaldehyd-Novolak,
10,60 Gt N-Vinylcarbazol,
0,24 Gt 2-(p-Dimethylamino-styryl)benzthiazol und
0,25 Gt Verbindung 2 in
84,60 Gt Butanon
wurde auf eine elektrolytisch aufgerauhte und anodisch oxydierte Aluminiumplatte aufgeschleudert und getrocknet. Das Trockenschichtgewicht betrug 1,5 g/m$^2$. Die Platte wurde wie im Anwendungsbeispiel 1 belichtet. Dabei schlug die Farbe der belichteten Bereiche von Gelb nach Orangerot um.

Die unbelichteten Schichtbereiche wurden durch Behandeln mit dem in Anwendungsbeispiel 1 angegebenen Entwickler ausgewaschen.

Anwendungsbeispiel 3

Eine Lösung von

1,60 Gt des Veresterungsprodukts aus 1 mol 2,3,4-Trihydroxybenzophenon und 3 mol 1,2-Naphthochinon-2-diazid-5-sulfonylchlorid,

6,60 Gt Novolak gemäß Anwendungsbeispiel 1,

0,22 Gt Verbindung 1 und

0,075 Gt Kristallviolett in

50,00 Gt Propylenglykolmonomethylether und

50,00 Gt Dimethylformamid

wurde auf eine elektrolytisch aufgerauhte und anodisch oxydierte Aluminiumplatte aufgeschleudert und getrocknet. Das Schichtgewicht betrug 2,3 $g/m^2$. Die Platte wurde 60 Sekunden mit der im Anwendungsbeispiel 1 angegebenen Lichtquelle unter einem Stufenkeil belichtet. Ein Bild mit gut sichtbarem Kontrast wurde erhalten. Die Platte wurde dann 60 Sekunden mit dem folgenden Entwickler behandelt:

8,5 g $Na_2SiO_3 \times 9\ H_2O$,

0,8 g NaOH,

1,5 g $Na_2B_4O_7 \times 10\ H_2O$,

89,2 g Wasser.

Es wurde ein positives Bild der Vorlage erhalten, in dem die Stufe 5 frei war.

Anwendungsbeispiel 4

Es wurde wie im Anwendungsbeispiel 3 gearbeitet, wobei aber anstelle der Verbindung 1 eine der Verbindungen 5 und 11 eingesetzt wurde. In beiden Fällen wurden Bilder mit gutem Belichtungskontrast und 5 frei entwickelten Stufen erhalten. Ähnliche Ergebnisse wurden erhalten, wenn eine der Verbindungen 8, 9 und 10 eingesetzt wurde.

Anwendungsbeispiel 5

Es wurde wie im Anwendungsbeispiel 2 gearbeitet, jedoch wurde anstelle des 2-(p-Dimethylaminostyryl)benzthiazols die gleiche Menge Sudangelb eingesetzt. Nach bildmäßiger Belichtung waren die belichteten Stellen rot gefärbt. Ähnliche Ergebnisse wurden mit den Verbindungen 6, 7, 3 oder 4 anstelle der Verbindung 2 erhalten.

Anwendungsbeispiel 6

Eine Lösung von

7,1 Gt Pentaerythrittriacrylat,

6,0 Gt Methylmethacrylat/Methacrylsäure-Copolymerem (Säurezahl 115),

0,15 Gt Verbindung 1 und

0,3 Gt eines Azofarbstoffs, erhalten durch Kuppeln von 2,4-Dinitro-6-chlor-benzoldiazoniumsalz mit 2-Methoxy-5-acetylamino-N-hydroxyethyl-N-cyanoethyl-anilin in

65 Gt 2-Methoxy-ethanol und

23 Gt Butylacetat

wurde auf eine elektrolytisch aufgerauhte und anodisch oxydierte Aluminiumplatte aufgeschleudert und getrocknet. Das Schichtgewicht betrug 1,5 $g/m^2$. Die lichtempfindliche Schicht wurde mit einer dünnen Polyvinylalkoholschicht überzogen.

Die Platte wurde wie im Anwendungsbeispiel 3 belichtet und 45 Sekunden in einer 1,5 %igen wäßrigen Natriummetasilikatlösung entwickelt. Es wurden 4 vollvernetzte Keilstufen erhalten.

Anwendungsbeispiel 7

Eine Lösung von

32,5 Gt einer 30,4 %igen Lösung eines Terpolymeren aus Styrol, n-Hexylmethacrylat und Methacrylsäure (10:60:30) mit der Säurezahl 190 in Butanon,

10,0 Gt des Diurethans aus 1 mol 2,2,4-Trimethylhexamethylendiisocyanat und 2 mol Hydroxyethylmethacrylat,

0,025 Gt Kristallviolettbase,

0,4 Gt Verbindung 11 und
0,4 Gt 2-Benzoylmethylen-3-methyl-naphtho[1,2-d]thiazolin in
70 Gt Dimethylformamid und
70 Gt 2-Methoxy-ethanol
wurde auf eine Platte aus elektrolytisch aufgerauhtem und anodisch oxydiertem Aluminium aufgeschleudert und getrocknet. Das Schichtgewicht betrug 1,6 $g/m^2$. Die lichtempfindliche Schicht wurde dann mit einer dünnen Polyvinylalkoholschicht überzogen. Die Platte wurde unter einem Stufenkeil 2 Sekunden mit der im Anwendungsbeispiel 1 angegebenen Lichtquelle belichtet, dann 1 Minute auf 100 °C erwärmt und innerhalb von 45 Sekunden mit der folgenden Lösung manuell entwickelt:
12 g Natriummetasilikat x 9 $H_2O$,
2,13 g Strontiumchlorid,
1,2 g nichtionogenes Netzmittel (Kokosölalkohol-Polyoxyethylenether mit ca. 8 Oxyethylengruppen) und
0,12 g Antischaummittel auf Silikonbasis in
4000 g Wasser.
Nach dem Entwickeln wurden 10 vollvernetzte Keilstufen erhalten.

**Patentansprüche**

1. Verfahren zur Herstellung einer Verbindung der Formel I

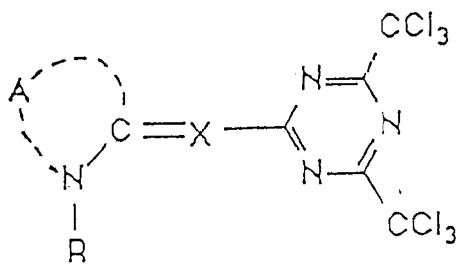

(I)

worin
A die zur Vervollständigung eines 5- oder 6-gliedrigen heterocyclischen Rings erforderlichen Ringglieder, der durch Alkyl- oder Alkoxygruppen mit 1 bis 6 Kohlenstoffatomen, Aryl- oder Aryloxygruppen mit 6 bis 10 Kohlenstoffatomen oder Halogenatome substituiert sein oder einen ankondensierten Benzol- oder Naphthalinkern tragen kann, der unsubstituiert oder durch gleichartige Gruppen wie der heterocyclische Ring substituiert ist,
R eine Alkyl-, Alkoxyalkyl- oder Halogenalkylgruppe mit 1 bis 6 Kohlenstoffatomen oder eine Aralkyl- oder Aryloxyalkylgruppe mit 7 bis 10 Kohlenstoffatomen und
X CH oder N
bedeutet,
dadurch gekennzeichnet, daß man 2,4,6-Tris-trichlormethyl-s-triazin mit dem Salz einer quaternären N-heterocyclischen Base der Formel III oder einem heterocyclischen Imin der Formel IV

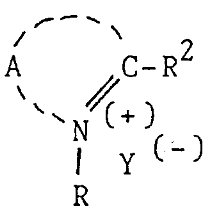

(III)

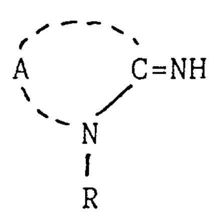

(IV)

umsetzt, wobei
$R^2$ eine Methyl- oder Aminogruppe und
$Y^{(-)}$ ein Anion ist.

2. Verfahren nach Anspruch 1, dadurch gekennzeichnet, daß man die Umsetzung in Gegenwart einer Base durchführt.

**3.** Verfahren nach Anspruch 2, dadurch gekennzeichnet, daß man als Base ein tertiäres Amin einsetzt.

**4.** Verfahren nach Anspruch 1, dadurch gekennzeichnet, daß man die Umsetzung in einem Lösemittel durchführt.

**5.** Verfahren nach Anspruch 1, dadurch gekennzeichnet, daß man die Umsetzung bei einer Temperatur von 20 bis 60 °C durchführt.

**Claims**

**1.** A process for the preparation of a compound of the formula I

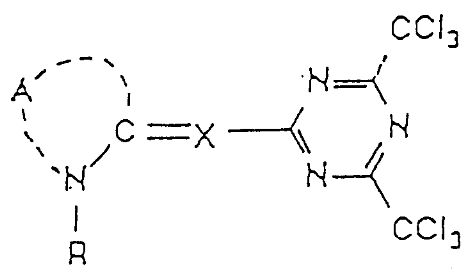

(I)

in which

A denotes the ring members required to complete a 5- or 6-membered heterocyclic ring which may be substituted by alkyl or alkoxy groups of 1 to 6 carbon atoms, aryl or aryloxy groups of 6 to 10 carbon atoms or by halogen atoms or may carry a fused-on benzene or naphthalene nucleus which is unsubstituted or substituted by the same kind of groups as the heterocyclic ring,

R denotes an alkyl, alkoxyalkyl or haloalkyl group of 1 to 6 carbon atoms or an aralkyl or aryloxyalkyl group of 7 to 10 carbon atoms, and

X denotes CH or N,

which comprises reacting 2,4,6-tris-trichloromethyl-s-triazine with the salt of a quaternary N-heterocyclic base of the formula III or with a heterocyclic imine of the formula IV

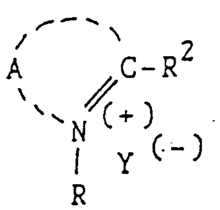

(III)

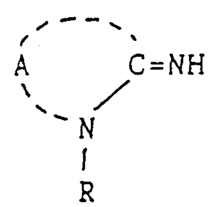

(IV)

where

$R^2$ is a methyl or amino group and

$Y^{(-)}$ is an anion.

**2.** A process as claimed in claim 1, wherein the reaction is carried out in the presence of a base.

**3.** A process as claimed in claim 2, wherein the base employed is a tertiary amine.

**4.** A process as claimed in claim 1, wherein the reaction is carried out in a solvent.

**5.** A process as claimed in claim 1, wherein the reaction is carried out at a temperature from 20 to 60 °C.

## Revendications

**1.** Procédé pour la préparation d'un composé de formule I

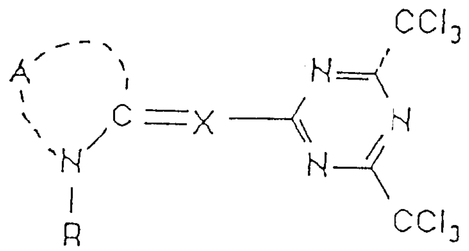

(I)

dans laquelle

A représente les chaînons de cycle requis pour compléter un hétérocycle à 5 ou 6 chaînons, qui peut être substitué par des groupes alkyle ou alcoxy ayant de 1 à 6 atomes de carbone, des groupes aryle ou aryloxy ayant de 6 à 10 atomes de carbone ou des atomes d'halogène, ou porter un noyau benzénique ou naphtalénique fusionné qui n'est pas substitué ou est substitué par des groupes de même nature que les substituants du hétérocycle,

R représente un groupe alkyle, alcoxyalkyle ou halogénoalkyle ayant de 1 à 6 atomes de carbone, ou un groupe aralkyle ou aryloxyalkyle ayant de 7 à 10 atomes de carbone, et

X représente CH ou N,

caractérisé en ce que l'on fait réagir de la 2,4,6-tris-trichlorométhyl-s-triazine avec le sel d'une base N-hétérocyclique quaternaire de formule III ou avec une imine hétérocyclique de formule IV

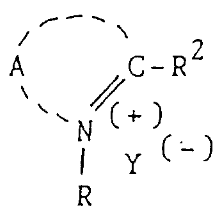

(III)

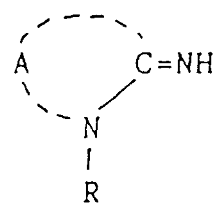

(IV)

$R^2$ étant le groupe méthyle ou amino, et

$Y^{(-)}$ étant un anion.

**2.** Procédé selon la revendication 1, caractérisé en ce que l'on effectue la réaction en présence d'une base.

**3.** Procédé selon la revendication 2, caractérisé en ce que, comme base, on utilise une amine tertiaire.

**4.** Procédé selon la revendication 1, caractérisé en ce que l'on effectue la réaction dans un solvant.

**5.** Procédé selon la revendication 1, caractérisé en ce que l'on effectue la réaction à une température de 20 à 60 °C.